# EUROPEAN PATENT SPECIFICATION

(11) **EP 3 141 277 B1**
(45) Date of publication and mention of the grant of the patent: **02.08.2017**
(21) Application number: 15184923.9
(22) Date of filing: 11.09.2015
(51) Int. Cl.: A61M 16/08, A61M 16/20, A61M 16/04, A61M 39/06, A61M 39/24

(54) **MEDICAL JOINT AND CHECK VALVE MODULE THEREOF**
MEDIZINISCHE VERBINDUNG UND RÜCKSCHLAGVENTILMODUL DAFÜR
RACCORD MÉDICAL ET SON MODULE DE CLAPET DE RETENUE

(43) Date of publication of application: 15.03.2017
(73) Proprietor: Chou, Jeng-Yu, Yilan County 263 (TW); Chen, Jiunn-Rong, Taichung City 408 (TW)
(72) Inventor: CHOU, Jeng-Yu, 263 Yilan County (TW); CHANG, Shi-Chuan, 263 Yilan County (TW); CHEN, Chin-Wei, 263 Yilan County (TW)
(74) Representative: Dantz, Jan Henning

(56) References cited:
- EP-A1- 1 269 925
- EP-A1- 2 604 203
- US-A1- 2005 161 048
- US-A1- 2010 024 818

## Description

### Technical Field

The present invention relates to a medical joint according to the preamble of claim 1.

### Background

A medical joint is extensively used in medical institutions and medical treatment. For example, if a patient does not breathe spontaneously, a mechanical ventilator is often used to assist in breath. The mechanical ventilator includes a breathing tube, and the medical joint is assembled between the breathing tube and a breathing mask on the patient's face. The medical joint communicates with the breathing tube and the breathing mask for delivering a gas generated by the mechanical ventilator to the breathing mask.

The present medical joint is often integrally formed with an extension tube extending therefrom. The extension tube is configured for insertion of a detector for detecting gas pressure, MDI, gas, and temperature, for insertion of a suction tube for removing sputum, or for other effects.

US 2005/0161048 A1 teaches a multiple seal port anesthesia adapter that is suitable for the simultaneous insertion of multiple devices or instruments into the lungs during ventilation of the patient. The anesthesia adapter comprises a first port sized to receive a first elongate instrument; a second port sized to receive a second elongate instrument; an endotrachial tube fitting configured to be coupled to an endotrachial tube, the endotrachial tube fitting fluidly coupled to the first and second ports; and a hose fitting configured to be coupled to a ventilator hose, the hose fitting fluidly coupled to the first and second ports. The first port seals around the first elongated instrument and the second port seals around the second elongate instrument.

EP 1269925 A1 teaches an access cannula for endoscopic surgery, including a cannula tube and a valve body. The valve body has a main body with a neck for releasably connecting one end of the cannula tube and has a valve which insures a tight closure of the cannula tube at the end, but allows instruments to be passed through the cannula tube. The main body is releasable from the cannula tube together with the valve body. EP 2604203 A1 teaches a sealing device for sealing a feedthrough for a medical instrument. The sealing device has a first sealing membrane made of elastic material and having a first slit, and a second sealing membrane made of elastic material and having a second slit. The two sealing membranes are set laminarily abutting on each other such that the respective slits are not parallel with each other and are unbranched. The thickness of first sealing membrane continuously or discontinuously decreases towards the first slit and the thickness of second sealing membrane continuously or discontinuously decreases towards the second slit.

However, the medical joint tends to have a problem of leaking an internal gas out from the extension tube of the medical joint, thereby affecting the gas pressure or concentration, or even worse, compromising the medical treatment to patient.

### SUMMARY

It is an object of the present invention to provide a medical joint, wherein a first slit and a second slit intersect each other so as to facilitate inserting a detector into the connection tube, via an intersection of the first slit and the second slit, to detect gas pressure, MDI, gas, temperature, or to facilitate inserting a suction tube for removing sputum, and to prevent the gas inside the connection tube from leaking out from the intersection of the first slit and the second slit, so as to achieve handiness and a gas-leak-proof effect of the medical joint.

Accordingly, the present invention provides a medical joint comprising the features of claim 1. The medical joint comprises: a connection tube, the connection tube including a first tube and a second tube extending from and communicating with the first tube, two ends of the first tube including a communicating opening and a first junction respectively, a distal end of the second tube including a second junction; and a check valve module, the check valve module being assembled to the communicating opening to seal the same, the check valve module including a first membrane and a second membrane disposed overlappingly, the first membrane including a first slit, the second membrane including a second slit, the first slit and the second slit intersecting each other. Particular embodiments of the invention have the following effects:
According to an embodiment of the invention, the medical joint further includes a first rotatable tube and a second rotatable tube, the first rotatable tube is connected to the first junction and rotatable with respect to the first tube, and the second rotatable tube is connected to the second junction and rotatable with respect to the second tube. Therefore, when the first rotatable tube or the second rotatable tube is connected to other external tubing, the position of the connection tube is maintained even if inadvertently touching, moving or rotating the external tubing, thereby enhancing the safety in using the medical joint. According to another embodiment of the invention, the first plate and the second plate are each a convex plate protruding in a direction away from the communicating opening, so that the first slit and the second slit are opened toward the same direction as the inserting direction of the detector or the suction tube, thereby guiding the detector or the suction tube to be inserted into the communicating opening, so as to promote convenience in using the medical joint.

### BRIEF DESCRIPTION OF THE DRAWINGS

The disclosure will become more fully understood from the detailed description and the drawings given herein below for illustration only, and thus does not limit the disclosure, wherein:
FIG. 1 is a perspective exploded view of a medical joint according to the present invention;
FIG. 2 is a perspective assembled view of the medical joint according to the present invention;
FIG. 3 is a cross-sectional view of the medical joint according to the present invention;
FIG. 4 is another cross-sectional view of the medical joint according to the present invention;
FIG. 5 is a schematic view showing a use state of the medical joint according to the present invention; and
FIG. 6 is another cross-sectional view of the medical joint according to another embodiment of the present invention.

### DETAILED DESCRIPTION

Detailed descriptions and technical contents of the present invention are illustrated below in conjunction with the accompany drawings. However, it is to be understood that the descriptions and the accompany drawings disclosed herein are merely illustrative and exemplary and not intended to limit the scope of the present invention.

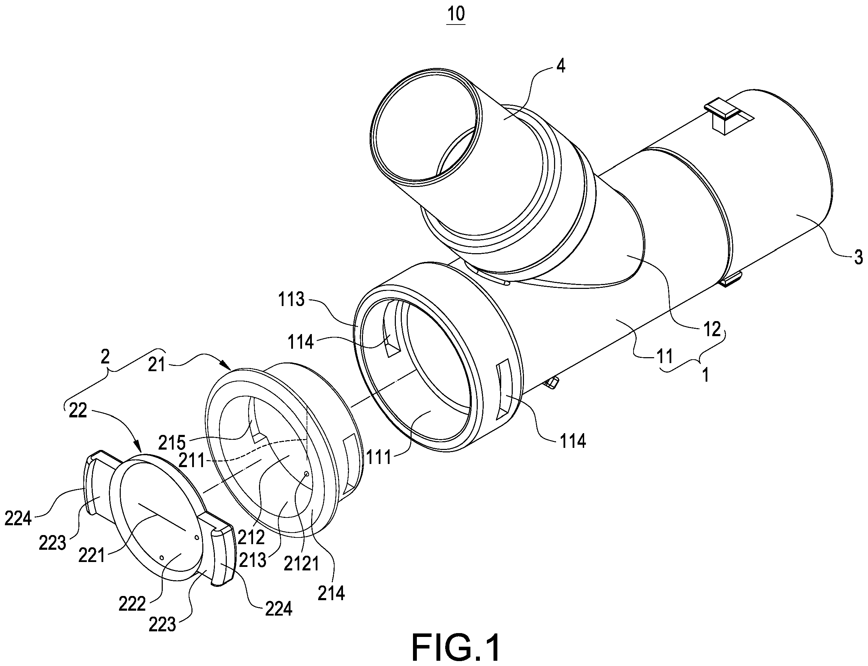

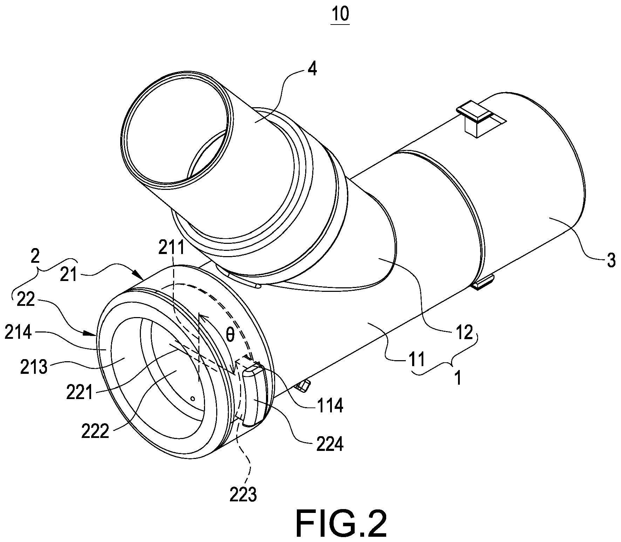

Referring to Figs. 1 to 5, the medical joint 10 includes a connection tube 1 and a check valve module 2. The check valve module 2 includes a first membrane 21 and a second membrane 22.

The connection tube 1 can be linear, Y-shaped, T-shaped, or of other suitable shape. In the present embodiment, the connection tube 1 is a Y-shaped tube as an example; however, the present invention is not limited thereto.

Referring to Figs. 1 to 4, the connection tube 1 includes a first tube 11 and a second tube 12 extending from and communicating with the first tube 11. Two ends of the first tube 11 include a communicating opening 111 and a first junction 112 respectively. A distal end of the second tube 12 includes a second junction 121. An included angle θ is formed between the first slit 211 and the second slit 221, and the included angle θ ranges from 30 to 150 degrees. In the best embodiment, the included angle θ is 90 degrees.

The first tube 11 forms a rim 113 at the communicating opening 111. A wall of the first tube 11 is disposed with two vents 114 opposite to each other. A first engagement ring 115 extends from an inner wall surface of the first tube 11.

Referring to Figs. 1 to 4, the check valve module 2 is assembled to the communicating opening 111 to seal the same. The check valve module 2 includes a first membrane 21 and a second membrane 22 disposed overlappingly. The first membrane 21 includes a first slit 211, the second membrane 22 includes a second slit 221, and the first slit 211 and the second slit 221 intersect each other.

In detail, the first membrane 21 includes a first plate 212 and an annular wall 213 extending from a periphery of the first plate 212. The annular wall 213 forms a stop ring 214 extending outwardly from one end of the annular wall 213 disposed away from the first plate 212. The annular wall 213 is disposed with two through holes 215 opposite to each other. The first slit 211 is formed on the first plate 212. The annular wall 213 is inserted in the first tube 11 and contacts the same. The stop ring 214 and the rim 113 abut each other for securement therebetween.

The second membrane 22 has a second plate 222 and two wings 223 extending from two sides of the second plate 222, a protrusion 224 extends from a distal end of each of the wings 223, the second slit 221 is formed on the second plate 222, the second plate 222 and the first plate 212 overlap and contact each other, the wings 223 pass through the respective through holes 215 and the respective vents 114 for securement, and the protrusion 224 and the first tube 11 engage each other.

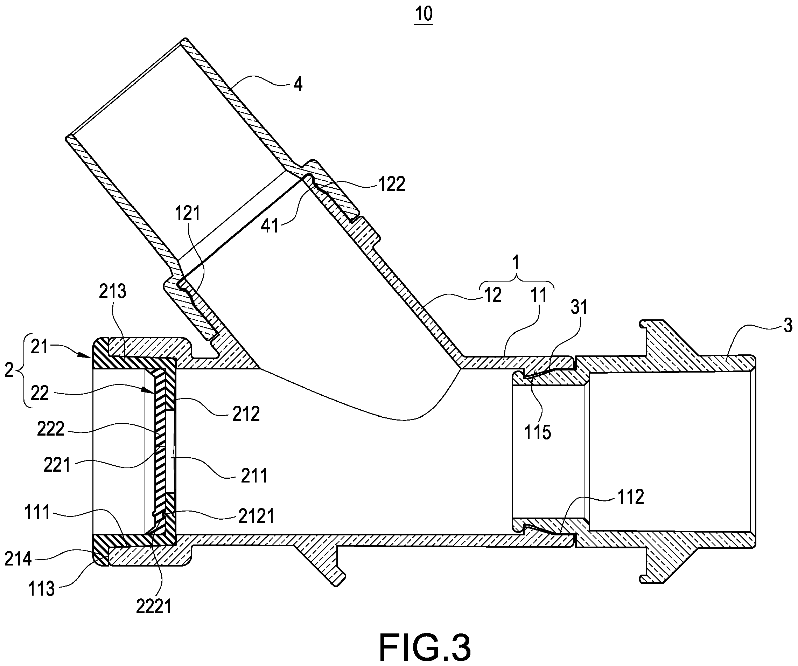

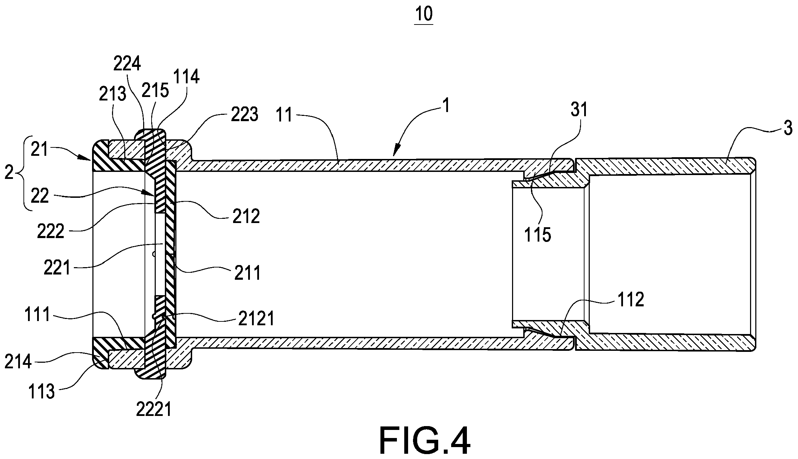

Furthermore, as shown in Figs. 3 and 4, the first plate 212 includes a plurality of protruding points 2121 extending therefrom, the second plate 222 is disposed with a plurality of trenches 2221, the protruding points 2121 are engaged with the trenches 2221 respectively, thereby preventing the first plate 212 from moving or rotating with respect to the second plate 222, so as to secure the first plate 212 in position.

Referring to Figs. 1 to 4, the medical joint 10 further comprises a first rotatable tube 3 and a second rotatable tube 4, the first rotatable tube 3 is connected to the first junction 112 and rotatable with respect to the first tube 11, and the second rotatable tube 4 is connected to the second junction 121 and rotatable with respect to the second tube 12.

In detail, a first annular recess 31 is formed on the exterior of the first rotatable tube 3, the first rotatable tube 3 is inserted in the first tube 11 via the first junction 112, the first engagement ring 115 is engaged with the first annular recess 31, and thereby the first rotatable tube 3 is rotatable with respect to the first tube 11.

A second annular recess 122 is formed on the exterior of the second tube 12, a first engagement ring 41 extends from the interior of the second rotatable tube 4, one end of the second rotatable tube 4 encloses the second junction 121 of the second tube 12, and the first engagement ring 41 is engaged with the second annular recess 122, and thereby the second rotatable tube 4 is rotatable with respect to the second tube 12.

Moreover, the first membrane 21 and the second membrane 22 consist of plastic, rubber, or other suitable materials. The first tube 11, the second tube 12, the first membrane 21, the second membrane 22, the first rotatable tube, or the second rotatable tube 4 can consist of or can be coated by an antibacterial material such as a silver-iron antibacterial material, so that the first tube 11, the second tube 12, the first membrane 21, the second membrane 22, the first rotatable tube 3, or the second rotatable tube 4 have an antibacterial property.

Referring to Figs. 3 and 4, the medical joint 10 of the present invention includes a connection tube 1 and the check valve module 2. The connection tube 1 includes a first tube 11 and a second tube 12 extending from and communicating with the first tube 11, two ends of the first tube 11 includes a communicating opening 111 and a first junction 112 respectively, and a distal end of the second tube 12 includes a second junction 121. The check valve module 2 is assembled to the communicating opening 111 to seal the same, the check valve module 2 includes a first membrane 21 and a second membrane 22 disposed overlappingly, the first membrane 21 includes a first slit 211, the second membrane 22 includes a second slit 221, and the first slit 211 and the second slit 221 intersect each other.

Referring to Figs. 3 and 4, in the check valve module 2, the first membrane 21 and the second membrane 22 are disposed overlappingly, the second membrane 22 includes a second slit 221, and the first slit 211 and the second slit 221 intersect each other.

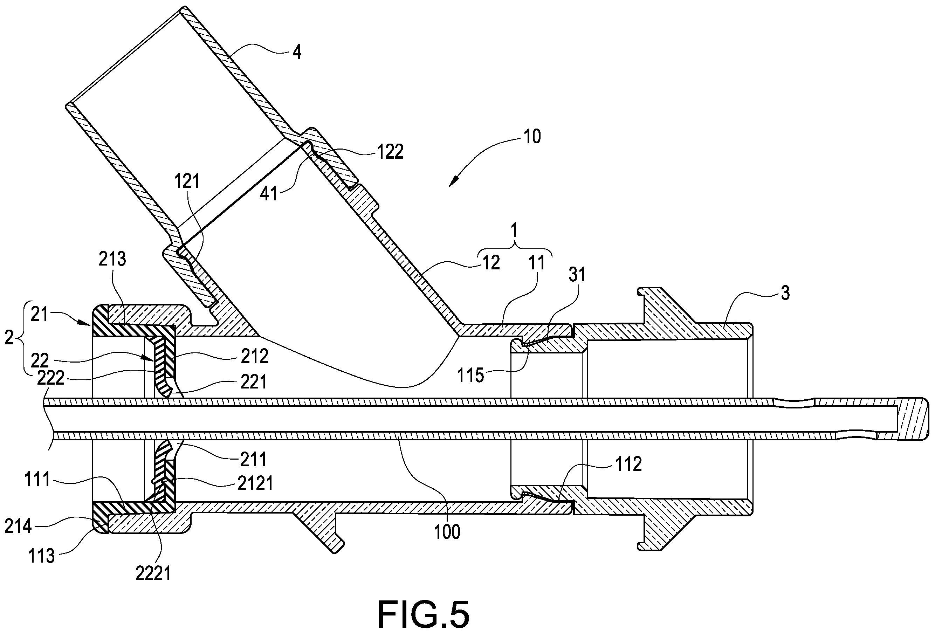

Referring to Fig. 5 which illustrates a use state of the medical joint 10 and the check valve module 2, the first membrane 21 is opened or closed via the first slit 211, and the second membrane 22 is opened or closed via the second slit 221, thereby facilitating inserting a detector into the connection tube 1 via an intersection of the first slit 211 and the second slit 221 so as to detect gas pressure, MDI, gas, temperature, or inserting a suction tube 100 to remove sputum. The first slit 211 and the second slit 221 intersect each other, so interference occurs between the open first membrane 21 and the open second membrane 22, thereby preventing the first membrane 21 and the second membrane 22 from opening too widely, thus avoiding a gas inside the connection tube 1 from leaking out from the intersection of the first slit 211 and the second slit 221, consequently achieving convenience and a gas-leak-proof property in using the medical joint 10 and the check valve module 2.

Referring to Figs. 1 to 5, the annular wall 213 is inserted in the first tube 11 and contacts the same, the stop ring 214 and the rim 113 abut each other for securement therebetween, the second plate 222 and the first plate 212 overlap and contact each other, the wings 223 pass through the respective through holes 215 and the respective vents 114 for securement, and the protrusion 224 and the first tube 11 engage each other, so that the first membrane 21 and the second membrane 22 are assembled securely to seal the communicating opening 111. Furthermore, the protruding points 2121 are engaged with the trenches 2221 respectively, thereby preventing the first plate 212 from moving or rotating with respect to the second plate 222, thus enhancing securement between the first membrane 21 and the second membrane 22.

Furthermore, referring to Figs. 1 to 5, the medical joint 10 further includes a first rotatable tube 3 and a second rotatable tube 4, the first rotatable tube 3 is connected to the first junction 112 and rotatable with respect to the first tube 11, and the second rotatable tube 4 is connected to the second junction 121 and rotatable with respect to the second tube 12. Therefore, when the first rotatable tube 3 or the second rotatable tube 4 is connected to other external tubing, the position of the connection tube 1 is maintained even if inadvertently touching, moving or rotating the external tubing, thereby enhancing the safety in using the medical joint 10.

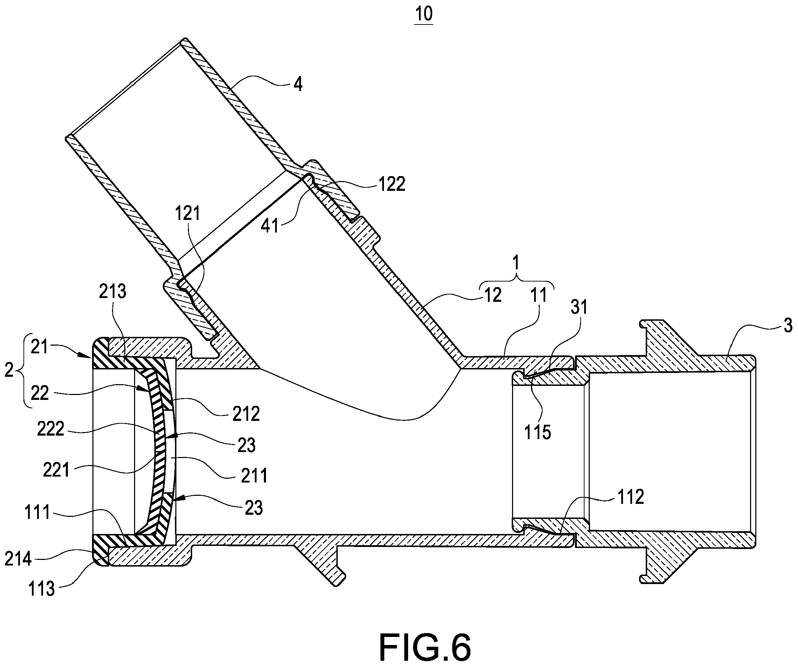

Fig. 6 illustrates the medical joint according to another embodiment of the present invention. The embodiment in Fig. 6 is similar to the embodiment in Figs. 1 to 5. The embodiment shown in Fig. 6 is different from the embodiment shown in Figs. 1 to 5 in that the first plate 212 and the second plate 222 are each a convex plate 23 protruding in a direction away from the communicating opening 111.

In detail, the first plate 212 and the second plate 222 are each a convex plate 23 protruding in a direction away from the communicating opening 111, so when the detector or the suction tube is inserted into the connection tube 1 via the communicating opening 111, the first slit 211 and the second slit 221 are opened toward the same direction as the inserting direction of the detector or the suction tube, thereby guiding the detector or the suction tube to be readily inserted into the communicating opening, so as to promote convenience in using the medical joint 10 and the check valve module 2.

Each convex plate 23 can be a plano-convex plate or a concavo-convex plate. In the present embodiment, each convex plate 23 is a concavo-convex plate; however, the present invention is not limited thereto.

## Claims

1. A medical joint, comprising:
a connection tube (1) including a first tube (11) and a second tube (12) extending from and communicating with the first tube (11), two ends of the first tube (11) including a communicating opening (111) and a first junction (112) respectively, a distal end of the second tube (12) including a second junction (121); and
a check valve module (2) assembled to the communicating opening (111) to seal the same, the check valve module (2) including a first membrane (21) and a second membrane (22) disposed overlappingly, the first membrane (21) including a first slit (211), the second membrane (22) including a second slit (221);
wherein the first slit (211) and the second slit (221) intersect each other,
**characterized in that** the first tube (11) forms a rim (113) at the communicating opening (111), the first membrane (21) includes a first plate (212) and an annular wall (213) extending from a periphery of the first plate (212), the annular wall (213) forms a stop ring (214) extending outwardly from one end of the annular wall (213) disposed away from the first plate (212), the first slit (211) is formed on the first plate (212), the annular wall (213) is inserted in the first tube (11) and contacts the same, the stop ring (214) and the rim (113) abut each other for securement therebetween, the first tube (11) includes two vents (114) opposite to each other, the annular wall (213) includes two through holes (215) opposite to each other, the second membrane (22) includes a second plate (222) and two wings (223) extending from two sides of the second plate (222), a protrusion (224) extends from a distal end of each of the wings (223), the second slit (221) is formed on the second plate (222), the second plate (222) and the first plate (212) overlap and contact each other, the wings (223) pass through the respective through holes (215) and the respective vents (114) for securement, and the protrusion (224) and the first tube (11) engage each other.

2. The medical joint of claim 1, wherein an included angle ( θ ) is formed between the first slit (211) and the second slit (221), and the included angle ( θ ) ranges from 30 to 150 degrees.

3. The medical joint of claim 1, wherein an included angle ( θ ) is formed between the first slit (211) and the second slit (221), and the included angle ( θ ) is 90 degrees.

4. The medical joint of claim 1, wherein the connection tube (1) is a Y-shaped tube or a T-shaped tube.

5. The medical joint of claim 1, wherein the first plate (212) includes a plurality of protruding points (2121) extending therefrom, the second plate (222) is disposed with a plurality of trenches (2221), the protruding points (2121) are engaged with the trenches (2221) respectively, and the first plate (212) and the second plate (222) are each a convex plate (23) protruding in a direction away from the communicating opening (111).

6. The medical joint of claim 1, further comprising a first rotatable tube (3) and a second rotatable tube (4), the first rotatable tube (3) being connected to the first junction (112) and rotatable with respect to the first tube (11), the second rotatable tube (4) being connected to the second junction (121) and rotatable with respect to the second tube (12).

7. The medical joint of claim 6, wherein a first engagement ring (115) extends from the interior of the first tube (11), a first annular recess (31) is formed on the exterior of the first rotatable tube (3), the first rotatable tube (3) is inserted in the first tube (11) via the first junction (112), the first engagement ring (115) is engaged with the first annular recess (31), a second annular recess (122) is formed on the exterior of the second tube (12), a first engagement ring (41) extends from the interior of the second rotatable tube (4), one end of the second rotatable tube (4) encloses the second junction (121) of the second tube (12), and the first engagement ring (41) is engaged with the second annular recess (122).

## Patentansprüche

1. Medizinische Verbindung, umfassend:
ein Verbindungsrohr (1), das ein erstes Rohr (11) und ein zweites Rohr (12) aufweist, das sich ausgehend von dem ersten Rohr (11) erstreckt und mit diesem kommuniziert, wobei zwei Enden des ersten Rohres (11) eine kommunizierende Öffnung (111) bzw. einen ersten Anschluss (112) aufweisen, wobei ein distales Ende des zweiten Rohres (12) einen zweiten Anschluss (121) aufweist; und
ein Rückschlagventilmodul (2), das an die kommunizierende Öffnung (111) angebaut ist, um diese abzudichten, wobei das Rückschlagventilmodul (2) eine erste Membran (21) und eine zweite Membran (22) aufweist, die überlappend angeordnet sind, wobei die erste Membran (21) einen ersten Schlitz (211) aufweist und die zweite Membran (22) einen zweiten Schlitz (221) aufweist;
wobei der erste Schlitz (211) und der zweite Schlitz (221) einander kreuzen,
**dadurch gekennzeichnet, dass** das erste Rohr (11) einen Kranz (113) an der kommunizierenden Öffnung (111) bildet, die erste Membran (21) eine erste Platte (212) und eine ringförmige Wand (213) aufweist, die sich ausgehend von einem Umfang der ersten Platte (212) erstreckt, die ringförmige Wand (213) einen Stoppring (214) bildet, der sich, von der ersten Platte (212) weggewandt angeordnet, von einem Ende der ringförmigen Wand (213) nach außen erstreckt, der erste Schlitz (211) in der ersten Platte (212) gebildet ist, die ringförmige Wand (213) in das erste Rohr (11) eingefügt ist und dieses berührt, der Stoppring (214) und der Kranz (113) für eine feste Verbindung zwischen ihnen aneinander anliegen, das erste Rohr (11) zwei Lüftungsöffnungen (114) aufweist, die einander gegenüberliegen, die ringförmige Wand (213) zwei Durchgangslöcher (215) aufweist, die einander gegenüberliegen, die zweite Membran (22) eine zweite Platte (222) und zwei Flügel (223) aufweist, die sich ausgehend von zwei Seiten der zweiten Platte (222) erstrecken, ein Vorsprung (224) sich ausgehend von einem distalen Ende von jedem der Flügel (223) erstreckt, der zweite Schlitz (221) in der zweiten Platte (222) gebildet ist, die zweite Platte (222) und die erste Platte (212) einander überlappen und berühren, die Flügel (223) zur Befestigung durch die jeweiligen Durchgangslöcher (215) und die jeweiligen Lüftungsöffnungen (114) hindurchgehen und der Vorsprung (224) und das erste Rohr (11) miteinander in Eingriff sind.

2. Medizinische Verbindung nach Anspruch 1, wobei ein eingeschlossener Winkel (θ) zwischen dem ersten Schlitz (211) und dem zweiten Schlitz (221) gebildet ist und der eingeschlossene Winkel (θ) 30 Grad bis 150 Grad beträgt.

3. Medizinische Verbindung nach Anspruch 1, wobei ein eingeschlossener Winkel (θ) zwischen dem ersten Schlitz (211) und dem zweiten Schlitz (221) gebildet ist und der eingeschlossene Winkel (θ) 90 Grad beträgt.

4. Medizinische Verbindung nach Anspruch 1, wobei das Verbindungsrohr (1) ein Y-förmiges Rohr oder ein T-förmiges Rohr ist.

5. Medizinische Verbindung nach Anspruch 1, wobei die erste Platte (212) eine Vielzahl von vorragenden Punkten (2121) aufweist, die sich ausgehend von ihr erstrecken, die zweite Platte (222) mit einer Vielzahl von Kerben (2221) versehen ist, die vorragenden Punkte (2121) jeweils in Eingriff mit den Kerben (2221) sind und die erste Platte (212) und die zweite Platte (222) jeweils eine konvexe Platte (23) sind, die in einer Richtung weg von der kommunizierenden Öffnung (111) vorragt.

6. Medizinische Verbindung nach Anspruch 1, ferner umfassend ein erstes drehbares Rohr (3) und ein zweites drehbares Rohr (4), wobei das erste drehbare Rohr (3) mit dem ersten Anschluss (112) verbunden ist und in Bezug auf das erste Rohr (11) drehbar ist, und wobei das zweite drehbare Rohr (4) mit dem zweiten Anschluss (121) verbunden ist und in Bezug auf das zweite Rohr (12) drehbar ist.

7. Medizinische Verbindung nach Anspruch 6, wobei ein erster Eingriffring (115) sich ausgehend von der Innenseite des ersten Rohres (11) erstreckt, eine erste ringförmige Vertiefung (31) an der Außenseite des ersten drehbaren Rohres (3) gebildet ist, das erste drehbare Rohr (3) durch den ersten Anschluss (112) in das erste Rohr (11) eingefügt ist, der erste Eingriffring (115) mit der ersten ringförmigen Vertiefung (31) in Eingriff ist, eine zweite ringförmige Vertiefung (122) an der Außenseite des zweiten Rohres (12) gebildet ist, ein erster Eingriffring (41) sich ausgehend von der Innenseite des zweiten drehbaren Rohres (4) erstreckt, ein Ende des zweiten drehbaren Rohres (4) den zweiten Anschluss (121) des zweiten Rohres (12) umschließt und der erste Eingriffring (41) mit der zweiten ringförmigen Vertiefung (122) in Eingriff ist.

## Revendications

1. Raccord médical, comprenant :
un tube de connexion (1) comprenant un premier tube (11) et un second tube (12) qui s'étend depuis et communique avec le premier tube (11), deux extrémités du premier tube (11) comprenant respectivement une ouverture de communication (111) et une première jonction (112), une extrémité distale du second tube (12) comprenant une seconde jonction (121) ; et
un module clapet de retenue (2) assemblé à l'ouverture de communication (111) pour rendre celle-ci étanche, le module clapet de retenue (2) comprenant une première membrane (21) et une seconde membrane (22) disposées de manière à se chevaucher, la première membrane (21) comprenant une première fente (211), la seconde membrane (22) comprenant une seconde fente (221) ;
dans lequel la première fente (211) et la seconde fente (221) se croisent,
**caractérisé en ce que** le premier tube (11) forme un rebord (113) à l'ouverture de communication (111), la première membrane (21) comprend une première plaque (212) et une paroi annulaire (213) s'étendant à partir d'une périphérie de la première plaque (212), la paroi annulaire (213) forme une bague d'arrêt (214) s'étendant vers l'extérieur à partir d'une extrémité de la paroi annulaire (213) disposée à l'écart de la première plaque (212), la première fente (211) est formée sur la première plaque (212), la paroi annulaire (213) est insérée dans le premier tube (11) et en contact avec celui-ci, la bague d'arrêt (214) et le rebord (113) sont en butée mutuelle pour la fixation entre eux, le premier tube (11) comprend deux évents (114) opposés l'un à l'autre, la paroi annulaire (213) comprend deux trous traversants (215) opposés l'un à l'autre, la seconde membrane (22) comprend une seconde plaque (222) et deux ailes (223) s'étendant depuis deux côtés de la seconde plaque (222), une saillie (224) s'étend depuis une extrémité distale de chacune des ailes (223), la seconde fente (221) est formée sur la seconde plaque (222), la seconde plaque (222) et la première plaque (212) se chevauchent et sont en contact l'une avec l'autre, les ailes (223) traversent les trous traversants (215) respectifs et les évents (114) respectifs pour la fixation, et la saillie (224) et le premier tube (11) sont mutuellement en prise.

2. Raccord médical selon la revendication 1, dans lequel un angle inclus (θ) est formé entre la première fente (211) et la seconde fente (221), et l'angle inclus (θ) est compris entre 30 et 150 degrés.

3. Raccord médical selon la revendication 1, dans lequel un angle inclus (θ) est formé entre la première fente (211) et la seconde fente (221), et l'angle inclus (θ) est de 90 degrés.

4. Raccord médical selon la revendication 1, dans lequel le tube de connexion (1) est un tube en forme de Y ou un tube en forme de T.

5. Raccord médical selon la revendication 1, dans lequel la première plaque (212) comprend une pluralité de points en saillie (2121) s'étendant à partir de celle-ci, la seconde plaque (222) est disposée avec une pluralité de tranchées (2221), les points en saillie (2121) sont respectivement en prise avec les tranchées (2221), et la première plaque (212) et la seconde plaque (222) sont chacune une plaque convexe (23) faisant saillie dans une direction l'éloignant de l'ouverture de communication (111).

6. Raccord médical selon la revendication 1, comprenant en outre un premier tube rotatif (3) et un second tube rotatif (4), le premier tube rotatif (3) étant relié à la première jonction (112) et pouvant tourner par rapport au premier tube (11), le second tube rotatif (4) étant relié à la seconde jonction (121) et pouvant tourner par rapport au second tube (12).

7. Raccord médical selon la revendication 6, dans lequel une première bague d'engagement (115) s'étend depuis l'intérieur du premier tube (11), un premier évidement annulaire (31) est formé sur l'extérieur du premier tube rotatif (3), le premier tube rotatif (3) est inséré dans le premier tube (11) par l'intermédiaire de la première jonction (112), la première bague d'engagement (115) est en prise avec le premier évidement annulaire (31), un second évidement annulaire (122) est formé sur l'extérieur du second tube (12), une première bague d'engagement (41) s'étend depuis l'intérieur du second tube rotatif (4), une extrémité du second tube rotatif (4) entoure la seconde jonction (121) du second tube (12) et la première bague d'engagement (41) est en prise avec le second évidement annulaire (122).
